# EUROPEAN PATENT APPLICATION

(11) **EP 4 174 867 A1**
(43) Date of publication of application: **03.05.2023**
(21) Application number: 21020531.6
(22) Date of filing: 27.10.2021
(51) Int. Cl.: G16H 10/60, G16H 15/00, G16H 20/10, G16H 40/60

(54) **SYSTEM AND METHOD FOR DELIVERING CLINICALLY RELEVANT DOSES OF NITRIC OXIDE (NO) TO A PATIENT**

(71) Applicant: Linde GmbH, 82049 Pullach (DE)
(72) Inventor: Thind, Mandip, 82049 Pullach (DE); Jacobsen, Brian, 82049 Pullach (DE)
(74) Representative: Reuß, Stephanie

(57) **Abstract**

System for delivering clinically relevant doses of nitric oxide (NO) to a patient comprising a nitric oxide delivery device (100) and a remote computing device (200) adapted for monitoring performance and/or maintenance data of the nitric oxide delivery device (100), wherein the remote computing device (200) is provided as a cloud hosted platform such that the performance and/or maintenance data can be uploaded from the nitric oxide delivery device (100) to the remote computing device (200), and processed information generated by the remote computing device (200) on the basis of the performance and/or maintenance data can be downloaded from the remote computing device (200) to the nitric oxide delivery device (100).

## Description

The present invention generally relates to a system for delivering clinically relevant doses of nitric oxide to a patient and a corresponding method.

Nitric oxide (NO) gas has the effect of dilating blood vessels in the lungs when it is inhaled, whereby oxygenation of the blood is enhanced, and also pulmonary hypertension can be reduced. Such NO therapy is performed over a certain amount of time, and after the end of the therapy, patients must be weaned off the NO in order to avoid certain negative consequences ("rebound effect").

Maintenance of devices for delivering doses of NO to a patient as well as keeping logs on NO doses administered to patients is of critical importance.

In the prior art, maintenance and log information is locally stored within such devices in a format that is, however, not interpretable by users of the device, typically clinicians. Such log files containing maintenance and log information must be sent to the manufacturer of the device for interpretation, for which the manufacturer typically uses in-house software solutions. Data in a form readable and/or interpretable for the user is then returned to the user. This procedure is cumbersome and error prone.

The present invention seeks to mitigate the problems as outlined above.

According to the present invention, there is provided a system for delivering clinically relevant doses of NO to a patient comprising the features of claim 1 and a corresponding method comprising the features of claim 8.

Advantageous embodiments of the invention are the subject matter of the dependent claims.

The invention provides a system for delivering clinically relevant doses of nitric oxide (NO) to a patient comprising at least one nitric oxide delivery device and a remote computing device adapted for monitoring performance and/or maintenance data of the at least one nitric oxide delivery device, wherein the remote computing device is provided as a cloud hosted platform such that the performance and/or maintenance data can be uploaded from the at least one nitric oxide delivery device to the remote computing device, and processed information generated by the remote computing device on the basis of the performance and/or maintenance data can be downloaded from the remote computing device to the at least one nitric oxide delivery device. The term "downloading to the at least one nitric oxide delivery device" shall also include downloading to an entity, of which the nitric oxide delivery device is a part, such as a hospital or a surgery. Hereby, handling of the nitric oxide delivery device can be simplified for a user, as all relevant data can for example be processed automatically by the remote computing device or by specialised staff trained to handle the remote computing device. For example, an essentially constant monitoring of the performance and handling of nitric oxide delivery devices is possible, such that machine faults or handling errors by a user of the nitric oxide delivery device can be more easily identified.

Advantageously, the remote computing device is adapted to receive further information from other sources than the at least one nitric oxide delivery device, this further information being usable in the generation of said processed information. By utilising such additional information, performance of the at least on nitric oxide delivery device can be further enhanced.

Such other sources can especially comprise further nitric oxide delivery devices and/or gas cylinder suppliers.

Advantageously, the remote computing device is also adapted to further utilise data from external databases.

One of the main advantages of the present invention is that data generated by the remote computing device and downloaded to the at least one nitric oxide delivery device is provided in a format readable by a user of the nitric oxide delivery device. This significantly reduces time and effort in connection with monitoring handling and performance data.

According to a preferred embodiment of the invention, the nitric oxide delivery device 100 comprises a dosing module and a monitoring module. A dosing module serves two dose appropriate amounts of nitric oxide to a patient. The dosing module is typically monitored by the monitoring module, which is advantageously connected to the remote computing device. The nitric oxide delivery device typically also comprises a display which can serve to provide an interaction for the user and which can show data. The nitric oxide delivery device also includes ports for connecting the dosing and monitoring tubing.

Advantageously, the nitric oxide delivery device is adapted to access and utilize the cloud hosted platform, which especially means that the features and benefits of the invention, such as being able to view and access historic therapy records, book service calls, general device performance summaries and recommendations for actions are accessible from the device itself, as well as, for example, from a third party computer, which can also be connected to the nitric oxide delivery device.

It is pointed out in this connection that, according to the invention, historic data from previous therapies (including a patient's own therapies, but also anonymized data from all patients) can be used to predict and recommend actions by a user during a therapy session. As an example, it is pointed out that it could automatically be detected that the user should start to wean the patient off nitric oxide and make suggestions to the user, i.e. the clinician, to begin lowering the NO dose over a period of time to optimally wean the patient.

In this connection, an additional or alternative variant could be that either the user selects the type or reason for therapy, for example, Covid-19 treatment, neo-natal conditions, cardiovascular conditions etc., and the device can use historic data from other devices to recommend and even automatically execute an optimum therapy program.

An additional implementation may comprise a benchmarking activity, in which the system shows, how the current therapy session maps against other historic therapy sessions of a similar nature.

The invention also provides a method for delivering clinically relevant doses of nitric oxide to a patient comprising the following steps: administering nitric oxide to a patient by means of a nitric oxide delivery device and monitoring performance and/or maintenance data of the nitric oxide delivery device by means of a remote computing device, wherein the remote computing device is provided as a cloud hosted platform such that the performance and/or maintenance data are uploaded from the nitric oxide delivery device to the remote computing device, and processed information generated by the remote computing device on the basis of the performance and/or maintenance data is downloaded from the remote computing device to the nitric oxide delivery device.

The invention will now be further described with reference to the accompanying figures. Herein,

Figure 1 shows a schematical view of a system according to a preferred embodiment of the system according to the invention, and

Figure 2 a flow diagram for illustrating a preferred embodiment of the method according to the invention.

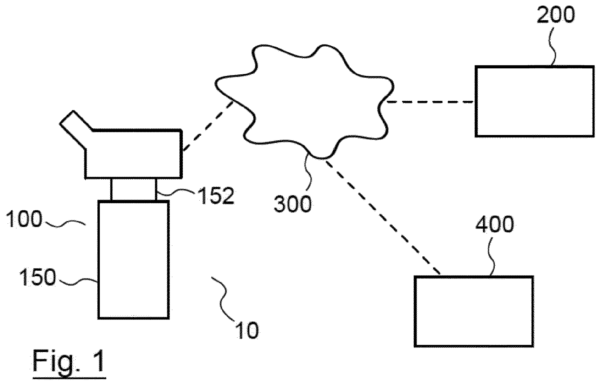

A preferred embodiment of a system according to the present invention is shown schematically in figure 1 and generally designated 10. The system comprises a nitric oxide delivery (NOD) device 100 for delivering clinically relevant doses of NO to a patient, as well as a remote computing device 200 for monitoring performance and/or maintenance data of the nitric oxide delivery device 100. Both devices 100 and 200 are shown in a purely schematical way. For example, gas outlet ports, tubings, pressure valves, displays and humidifiers, which constitute typical components of a NOD device, as well as a computer or server architecture, which is typically included in a remote computing device, are not shown.

Devices 100 and 200 are connected via an internet connection, i.e. are cloud based, as schematically indicated by cloud 300.

Nitric oxide delivery device 100 is connected to a gas cylinder 150 containing nitrogen or nitric oxide or any other suitable gas mixture for delivering clinically relevant doses of NO to a patient. The doses delivered can be set by a user by means of a control module 152.

The system is provided as a cloud hosted system which allows the user to use for example a PC, tablet or phone to upload data, typically provided in log files, from NOD device 100 to remote computing device 200, so that such log files can be processed in order to be translated and interpreted to produce meaningful information for the user. Such meaningful information can for example comprise the start time of a therapy, the end time of a therapy, a graph or table plotting the NO value set by a user, typically a clinician, each time it is changed, along with time and date data as to when such changes takes place.

The log files can include patient or therapy related data as well as device related data, such as maintenance data.

Advantageously, as patient or therapy related data, the data can include measurements recorded by NOD device 100, such as measurements of administered NO, NO₂ and/or O₂, plotted on a graph over time, or listed in a table, including when these measurements exceeded certain thresholds, such as alarm thresholds. Alert and alarm events which occurred during a therapy can be listed in a table or overlaid on a timeline.

Typical examples of maintenance data are data relating to time and date of calibrations or calibration attempts, including if such attempts were successful, or the results of such calibrations. Further maintenance data can include a log of which cylinder connector was used, and when a cylinder 150 was exchanged.

By combining such data with data from external databases, which are schematically designated 400 in figure 1, such data including device tracking tools, gas cylinder shipment data, patient data records and service records, remote computing device 200 can use algorithms for example to display general usage data about a specific NOD device 100, such as uptime/utilisation, average NO values, for example to predict overall gas consumption of a device 100.

Be it noted that although only one NOD device 100 is shown in figure 1, this is meant to comprise situations in which a number of such devices 100 are provided either at the same location, for example the same hospital unit, or at locations remote from one another. By comparing log files received from different NOD devices 100, for example malfunctioning NOD devices, or even specific malfunctions, can be identified.

Maintenance data can, for example, also include track service intervals, in order to alert users when a service is due and to facilitate booking of a service slot for maintenance work.

Also, commonly occurring alarms can be monitored. Furthermore, customer services can be provided, such as training or retraining opportunities. This would, for example, allow the manufacturer of devices 100 or devices 200 to update and focus training material to areas in which frequent alarms or other issues are encountered.

The data, i.e. log files, can be extracted from NOD device 100 in a number of ways. For example, by connecting a USB memory stick to a USB port of NOD device 100, the device can automatically transfer data or the log file to the memory stick. The user can then insert the USD stick into a PC, tablet or phone and upload the file, via cloud 300, to remote computing device 200 for further processing or interpretation.

NOD device 100 can, alternatively or in addition, be equipped with a Bluetooth or other short range wireless communications device, and an associated "hub" PC or device in close proximity to NOD device can be provided, such that log files from NOD device 100 can be wirelessly transferred to said associated hub PC, and then uploaded via the cloud as described above. In this case, no user intervention is required, whereby a regular and automatic upload of data can be provided.

As a further example, it is possible to equip NOD device 100 with a 2G, 3G, 4G or other cellular module, which can communicate with a mobile phone network, such that the data (log files) can be uploaded from NOD device automatically and regularly. A further benefit of such a module is that, in addition to the seamless upload of log file data, the device can be geo-located, for example, when it is moved outside of the site of a user (for example, during shipment or when used in an ambulance).

The invention provides numerous benefits for users. For example, interpretation of log files of an NOD device 100 is possible without having to provide software locally, i.e. on or in the vicinity of such a device. Users can create a custom report including their own notes and references, and export this report, together with the log file, to their desired or preferred record management system, i.e. the entity which provides remote computing device 200.

Hereby, not only a user, but also the entity providing remote computing device 200, can obtain a general overview of a NOD device 100 overtime, information available, including uptime, utilisation, common therapy settings and an estimated gas consumption. Based on this data, for example an optimised re-ordering plan for gas, consumables and service activities can be proposed to the user/customer without any human intervention. Frequently occurring alarm events can be flagged to the user, thus to indicate areas or issues where an additional training may be expedient.

On the other hand, a record management provider hosting remote computing device 200 receives an overview a customer activity. Hereby, the overall market can be monitored, and also individual user or customer activity can be monitored over time. It is, for example, possible to identify infrequent uses or abnormal uses, or frequently according alarm events so that for example account managers can intervene in order to enhance the customer experience. Also, market-level estimations on gas and consumable consumption can be generated for forward inventory planning. The invention thus provides an additional method of tracking devices, for example in addition to barcode scanning.

With the invention, it is possible to provide automated and regular maintenance data relating to NOD devices 100, especially maintenance warnings, to users. Hereby, it can for example be avoided that a maintenance interval expires during a current therapy. As users usually have multiple devices at their disposal, the system can then recommend another device that a user should use. Especially for the manufacturers of NOD devices 100 and distributors of the nitric oxide gas, which is classed as a pharmaceutical, capturing customer complaints and recording incidents and failures in the market place is of great importance, for example due to legal requirements. Automated readout of log files and corresponding regular provision of information relating to performance and/or maintenance is highly advantageous in this connection.

Also, by collecting usage data, additional services could be provided to users, such as the automatic dispatch of spares and consumables, including collecting empty gas cylinders and sending out full ones when there are sufficient indications that they are running low on gas and an exchange would be expedient.

Overtime, sensors utilised within NOD devices 100, such as NO and NO₂ sensors, degrade in performance. This is usually corrected with a calibration which the user is asked to perform from time to time. However, as the sensors age, even this calibration cannot always compensate for their decline in performance. Eventually such sensors must be replaced. Currently, sensors are replaced at set intervals, not taking into consideration their age or their actual status. By utilising the invention, i.e. regularly uploading usage data contained in log files to remote computing device 200, measured NO and NO₂ values stored can be analysed, and actual sensor performance, just as the performance of other relevant components, can be determined. Hereby, it can be ascertained when performance is so poor that the sensor or component must be replaced. An enhanced efficiency in connection with providing replacement sensors or components can thus be realised.

When a NOD device 100 is taken into use, it does not yet know what ventilator or circuit it will be attached to. It also does not feature separate dosing and monitoring modules. This means that, when a device is first taken into use, high doses of nitric oxide must be provided, wherein these high doses are quickly corrected when the presence of nitric oxide is then actually measured. Utilising the present invention, the device logs its behaviour throughout the therapy session. For example, before taking a device into use, the user could be asked, via the cloud, to provide more details about the ventilator and the circuit which are used, such that the initial nitric oxide doses could be provided at a lower level.

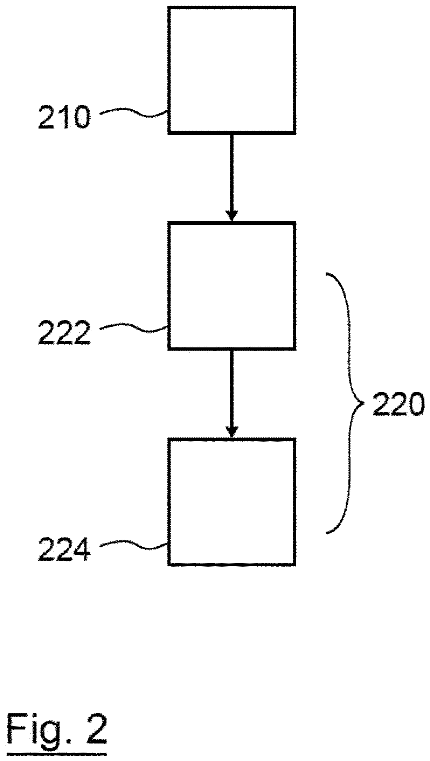

A preferred embodiment of the method according to the invention will now be described in connection with Figure 2.

In a first step 210 clinically relevant doses of nitric oxide (NO) are administered to a patient to a patient by means of a nitric oxide delivery device 100.

In a subsequent step 220 performance and/or maintenance data of the nitric oxide delivery device 100 are monitored by means of a remote computing device 200.

The remote computing device 200 is provided as a cloud hosted platform such that the step 220 of monitoring the performance and/or maintenance data comprises a step of uploading 222 the performance and/or maintenance data from the nitric oxide delivery device 100 to the remote computing device 200, and a step 224 of downloading processed information generated by the remote computing device on the basis of the performance and/or maintenance data from the remote computing device 200 to the nitric oxide delivery device 100.

## Claims

1. System for delivering clinically relevant doses of nitric oxide (NO) to a patient comprising a nitric oxide delivery device (100) and a remote computing device (200) adapted for monitoring performance and/or maintenance data of the nitric oxide delivery device (100), wherein the remote computing device (200) is provided as a cloud hosted platform such that the performance and/or maintenance data can be uploaded from the nitric oxide delivery device (100) to the remote computing device (200), and processed information generated by the remote computing device (200) on the basis of the performance and/or maintenance data can be downloaded from the remote computing device (200) to the nitric oxide delivery device (100).

2. System according to claim 1, wherein the remote computing device (200) is adapted to receive further information from other sources than the nitric oxide delivery device (100), this further information being usable in the generation of said processed information.

3. System according to claim 2, wherein said other sources comprise further nitric oxide delivery devices or gas cylinder suppliers.

4. System according to any one of the preceding claims, wherein the remote computing device (200) is adapted to further utilise data from external databases.

5. System according to any one of the preceding claims, wherein the data generated by the remote computing device (200) and downloaded to the nitric oxide delivery device (100) is provided in a format readable by a user of the nitric oxide delivery device (100).

6. System according to any one of the preceding claims, wherein the nitric oxide delivery device (100) comprises a dosing module and a monitoring module.

7. System according to any one of the preceding claims, wherein the nitric oxide delivery device (100) is provided such that it can access and utilize information from the remote computing device (200), especially the processed information generated by the remote computing device (200).

8. Method for delivering clinically relevant doses of nitric oxide (NO) to a patient comprising the following steps: administering (210) nitric oxide to a patient by means of a nitric oxide delivery device (100) and monitoring (220) performance and/or maintenance data of the nitric oxide delivery device (100) by means of a remote computing device (200), wherein the remote computing device (200) is provided as a cloud hosted platform such that the step (210) of monitoring the performance and/or maintenance data comprises uploading (222) the performance and/or maintenance data from the nitric oxide delivery device (100) to the remote computing device (200), and downloading (224) processed information generated by the remote computing device (200) on the basis of the performance and/or maintenance data from the remote computing device (200) to the nitric oxide delivery device (100).
